(19) Europäisches Patentamt / European Patent Office / Office européen des brevets

(11) **EP 2 513 036 B1**

(12) **EUROPÄISCHE PATENTSCHRIFT**

(45) Veröffentlichungstag und Bekanntmachung des Hinweises auf die Patenterteilung:
**17.04.2019 Patentblatt 2019/16**

(21) Anmeldenummer: **10787811.8**

(22) Anmeldetag: **14.12.2010**

(51) Int Cl.:
***C07C 209/26*** (2006.01) ***C07C 213/00*** (2006.01)
***C07C 211/10*** (2006.01) ***C07C 215/08*** (2006.01)

(86) Internationale Anmeldenummer:
**PCT/EP2010/069642**

(87) Internationale Veröffentlichungsnummer:
**WO 2011/082994 (14.07.2011 Gazette 2011/28)**

(54) **UMSETZUNG VON GLYKOLALDEHYD MIT EINEM AMINIERUNGSMITTEL**

REACTING GLYCOLALDEHYDE WITH AN AMINIATING AGENT

CONVERSION DE GLYCOLALDÉHYDE AVEC UN AGENT D'AMINATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2009 EP 09179710**

(43) Veröffentlichungstag der Anmeldung:
**24.10.2012 Patentblatt 2012/43**

(73) Patentinhaber: **BASF SE**
**67056 Ludwigshafen am Rhein (DE)**

(72) Erfinder:
- **MÄGERLEIN, Wolfgang**
  **68165 Mannheim (DE)**
- **MELDER, Johann-Peter**
  **67459 Böhl-Iggelheim (DE)**
- **PASTRE, Jörg**
  **64625 Bensheim (DE)**
- **EBERHARDT, Jan**
  **68167 Mannheim (DE)**
- **KRUG, Thomas**
  **67550 Worms (DE)**
- **KREITSCHMANN, Mirko**
  **68161 Mannheim (DE)**

(74) Vertreter: **BASF IP Association**
**BASF SE**
**G-FLP-C006**
**67056 Ludwigshafen (DE)**

(56) Entgegenhaltungen:
**EP-A1- 0 737 514** **DE-A1- 4 400 591**
**US-A- 6 147 261** **US-B1- 6 534 441**

- **DUANE F. MORROW ET AL: "Antifertility activity of some .beta.-amino alcohols", JOURNAL OF MEDICINAL CHEMISTRY, Bd. 16, Nr. 6, 1. Juni 1973 (1973-06-01), Seiten 736-739, XP55000068, ISSN: 0022-2623, DOI: 10.1021/jm00264a046**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 513 036 B1

Printed by Jouve, 75001 PARIS (FR)

## Beschreibung

Die vorliegende Erfindung betrifft die Umsetzung von Glykolaldehyd mit einem Aminierungsmittel.

Die Umsetzung von hydroxy-substituierten Aldehyden mit Aminierungsmitteln, wie Ammoniak, ist aus dem Stand der Technik bekannt.

Aus Houben/Weyl, Methoden der organischen Chemie, Bd. XI/1, 4. Aufl., Georg Thieme Verlag Stuttgart, 1957, Seite 602 bis 648 sind Nickel- oder Kobaltkatalysatoren, vor allem in Form ihrer Raney-Metalle, sowie Katalysatoren der Pt-Gruppe zur hydrierenden Aminierung von hydroxy-substituierten Aldehyden bzw. Ketonen bekannt.

US 4,153,581 betrifft die Aminierung von Alkoholen, Aldehyden oder Ketonen mittels spezifischer Co/Cu - Katalysatoren, die Fe, Zn und/oder Zr enthalten während US 4,152,353 die Aminierung von Alkoholen, Aldehyden oder Ketonen mittels spezifischer Ni/Cu - Katalysatoren, die Fe, Zn und/oder Zr enthalten, betrifft.

WO 03/076386 A und EP 1 431 271 A1 beschreiben Verfahren zur Herstellung von Aminen aus Alkoholen bzw. Aldehyden oder Ketonen und Stickstoffverbindungen unter Verwendung eines Katalysators, dessen katalytisch aktive Masse 22-40 Gew.-% (bzw. 22-45 Gew.-%) sauerstoffhaltige Verbindungen des Zirkoniums, 1-30 Gew.-% sauerstoffhaltige Verbindungen des Kupfers und jeweils 15-50 Gew.-% (bzw. 5-50 Gew.-%) sauerstoffhaltige Verbindungen des Nickels und Kobalts enthält.

Fünf Patentanmeldungen (WO-A-2008/006750, WO-A-2008/006748, WO-A-2008/006752, WO-A-2008/006749, WO-A- 2008/006754) betreffen bestimmte dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Die in den oben genannten Anmeldungen beschriebenen Katalysatoren enthalten 10 bis 50 Gew.-% Co.

DE-A-211 82 83 betrifft ein Verfahren zur Herstellung sekundärer oder tertiärer aliphatischer oder cycloaliphatischer Amine durch Umsetzung einer aliphatischen oder cycloaliphatischen Carbonylverbindung mit Ammoniak unter Einsatz eines Pd/Ag-Festbettkatalysators. Trägermaterial ist insbesondere $SiO_2$.

EP-A2-312 253 beschreibt die Verwendung spezifischer Kupferkatalysatoren bei der Herstellung von N-substituierten Aminen aus Alkoholen oder Aldehyden.

In der DE 3609978 A1 wird ein Verfahren zur Herstellung von Hydroxyaminen aus Hydroxycarbonylverbindungen beschrieben, wobei auch Glykolaldehyd generisch eingeschlossen ist. Dabei wird in einem zweistufigen Verfahren die Carbonylverbindung zunächst mit dem Aminierungsmittel umgesetzt, bevor in einem zweiten Schritt das erhaltene Imin mit Wasserstoff zum entsprechenden Amin hydriert wird.

In den oben genannten Referenzen ist zwar generisch die Umsetzung von Aldehyden, u.a. auch Hydroxysubsituierten Aldehyden miterfasst, die Umsetzung von Glykolaldehyd ist jedoch nicht explizit offenbart.

Die US 6,534,441 beschreibt ein Verfahren zur reduktiven Aminierung von niederen aliphatischen Alkanderivaten unter Verwendung eines Nickel/Rhenium-Katalysators. Als ein möglicher Einsatzstoff wird in der Beschreibung Glykolaldehyd genannt. Der Nickel/Rhenium-Katalysator wird durch Imprägnierung eines Aluminosilikat-Katalysatorträgers mit einer Lösung eines Nickel- und eines Rheniumsalzes hergestellt. Im Anschluss kann eine Calcinierung des so erhaltenen Katalysators erfolgen. Das Gewichtsverhältnis von Nickel zu Rhenium liegt im Bereich von 1:1 bis 200:1. Vor dem Einsatz kann der calcinierte Katalysator aktiviert bzw. reduziert werden. Die Umsetzung der Alkanderivate erfolgt in der Regel im Bereich von 125 bis 350°C, einem Druck von ca. 25 bis 350 bar. Die Reaktion wird in der Regel kontinuierlich durchgeführt. Der Einsatz eines Lösungsmittels in die Reaktion wird nicht erwähnt.

In der deutschen Patentanmeldung DE-A1-4400591 wird ein Verfahren zur Herstellung von Aminoalkoholen durch Umsetzung von Hydroxycarbonylverbindungen mit Wasserstoff und einem Aminierungsmittel bei Temperaturen von 0 bis 300°C, Drücken von 1 bis 400 bar an einem Katalysator beschrieben, der 50 bis 100 Gew.-% Ruthenium enthält. Die Umsetzung kann in Abwesenheit oder Gegenwart eines inerten Lösungsmittels durchgeführt werden. Als möglicher Einsatzstoff wird u.a. auch Glycolaldehyd genannt. Der DE-A1-4400591 ist ebenfalls zu entnehmen, dass die Aminierung von Hydroxyaldehyden zu verfäbten Produkten führt. DE-A1-4400591 lehrt deshalb den Einsatz von Katalysatoren, die das Edelmetall Ruthenium in hoher Konzentration enthalten.

Die Umsetzung von Hydroxyalkanalen zu Diaminen in Gegenwart von Ammoniak und Wasserstoff in Gegenwart von Katalysatoren die Nickel oder Cobalt enthalten, wird in der US 6,147,261 offenbart. Die Umsetzung der Hydroxyalkanale zu den entsprechenden Diaminen kann in einer einstufigen Reaktion bei Temperaturen von 140 bis 180°C und einem Druck von mindestens 35 bar erfolgen. Die Umsetzung von 3-Hydroxypropionaldehyd kann auch in einer zweistufigen Reaktion erfolgen, wobei in der ersten Verfahrensstufe 3-Aminopropanol gebildet wird, welches in einer nachfolgenden zweiten Stufe zu Propylendiamin umgesetzt wird. Die erste Stufe erfolgt bei Temperaturen von mehr als 50°C, vorzugsweise von 100 bis 150°C und einem Druck von mehr als 35 bar. Die zweite Stufe wird bei einer Temperatur von 140 bis 200°C durchgeführt. In der ersten Stufe beträgt die Ausbeute von 3-Aminopropanol maximal 84%. Der Umsatz von Hydroxypropanal ist im wesentlich quantitativ. Die Umsetzung kann optional in Gegenwart eines Lösungsmittels, wie Wasser oder höheren Kohlenwasserstoffen, durchgeführt werden. Die Katalysatoren werden vor ihrem Einsatz nicht aktiviert oder reduziert.

In der US 6,147,261 wird gelehrt, dass Hydroxypropioaldehyd sehr reaktiv ist und zu Oligomerisierung und Polymerisation neigt. Die Umsetzung von Hydroxypropioaldehyd mit Ammoniak wird deshalb bevorzugt in Gegenwart eines Lösungsmittels durchgeführt.

In der US 6,147,261, DE-A1-4400591 und der US 6,534,441 wird zwar der Einsatz von Gylkolaldehyd als Einsatzstoff in einer Umsetzung mit einem Aminierungsmittel genannt, die konkrete, durch Beispiele belegte, Umsetzung wurde allerdings nicht beschrieben.

Die Umsetzung von Glykolaldehyd mit einem Aminierungsmittel in Gegenwart eines $PtO_2$ Katalysators und Wasserstoff in Ethanol wird in D.F. Morrow, J. Med. Chem., 16 (1973) 736-739 beschrieben.

In der US 6,147,261 wird ein Verfahren beschrieben, in dem Glykolaldehyd mit einem Amin, in Gegenwart eines Katalysators der Gruppe VIII und Wasserstoff, umgesetzt wird.

Im Gegensatz zu den höheren Homologen neigt Glykolaldehyd noch stärker zur Bildung des Dimers, in diesem Fall 2,5-Dihydroxy-1,4-dioxan, einer aufgrund seiner thermodynamischen Stabilität bevorzugt gebildeten Sechsring-Verbindung. So existiert Glykolaldehyd im Festzustand ausschließlich in dimerer Form (A. Beeby, D. B. H. Mohammed, J. R. Sodeau, J. Am. Chem. Soc., 109 (1987) 857-861).

In nicht wässriger Lösung liegt Glykolaldehyd ebenfalls nur in geringen Mengen als Monomer vor (<6%). Der Hauptanteil ist eine Gleichgewichtsmischung aus 5- und 6-Ring-Dimeren (Loc. cit). In wässriger Lösung existiert Glykolaldehyd hauptsächlich in Form des Hydrats (ca. 70%) und in dimerer Form (ca. 26%). Nur ca. 4% des Glykolaldehyds liegt in der Gleichgewichtsmischung in monomerer Form vor (Loc. cit). Somit liegt die zu aminierende Aldehydfunktion des monomeren Glykolaldehyds in Lösung nur untergeordnet vor. Weiterhin besteht bei Glykoladehyd als CH-acide Verbindung eine sehr hohe Tendenz in einer Aldolkondensation zu höheren Zuckeralkoholen zu polymerisieren, wobei stark verfärbte Produkte entstehen und die Ausbeute an Zielprodukt stark verringert wird.

Die Aufgabe der vorliegenden Erfindung bestand deshalb darin, ein Verfahren zur Aminierung von Glykolaldehyd zu entwickeln, das einen hohen Umsatz von Glykolaldehyd und die Bildung der Produkte, insbesondere von Ethanolamin (MEOA) und Ethylendiamin (EDA), in hoher Ausbeute und Selektivität ermöglicht. Insbesondere sollte die Bildung des Nebenprodukts Piperazin verringert werden, da dieses nur schlecht von MEOA bzw. EDA abgetrennt werden kann und in vielen Anwendungen störend wirkt. Zudem sollten die Umsetzungsprodukte in einer hohen Reinheit anfallen. Diese Ziele sollten unter der Prämisse erreicht werden, dass ein Katalysator eingesetzt werden kann, der weitestgehend frei von Edelmetallen ist, um die Stoffkosten des Verfahrens zu senken. Die Verwendung von edelmetallhaltigen Katalysatoren führt nämlich zu einer starken Erhöhung der Katalysatoreinsatzkosten, die sich auf die Wirtschaftlichkeit des Verfahrens auswirken. In Zukunft ist mit einer drastischen Verknappung von Rohstoffen zu rechnen, so dass erwartet werden kann, dass die Preise für Edelmetalle noch weiter ansteigen.

Weiterhin sollte der Katalysator eine hohe mechanische und chemische Beständigkeit aufweisen, um hohe Standzeiten zu gewährleisten.

Eine weitere Aufgabe der vorliegenden Erfindung bestand darin, dass sich möglichst geringe Mengen an Metallen, wie z. B. Aluminium im Fall von Skelett-Katalysatoren oder alkalische Promotoren wie Lithium, aus dem Katalysator herauslösen, da dies zu nachlassender Stabilität und Deaktivierung des Katalysators führt. Aluminate, die sich aus dem herausgelösten Aluminium unter basischen Bedingungen bilden, können als feste Rückstände zu Verstopfungen und Ablagerungen führen und die Zersetzung von Wertprodukt bewirken.

Die Aufgabe wurde gelöst durch ein Verfahren zur Umsetzung von Glykolaldehyd mit einem Aminierungsmittel in Gegenwart von Wasserstoff und eines Katalysators, wobei man den Katalysator durch Reduktion eines Katalysatorvorläufers oder durch Reduktion eines passivierten Katalysators aktiviert, dadurch gekennzeichnet, dass die Umsetzung in Gegenwart eines Lösungsmittels erfolgt und dass der Glykolaldehyd mit dem aktivierten Katalysator in Kontakt gebracht wird.

In das erfindungsgemäße Verfahren wird Glykolaldehyd eingesetzt. Glykolaldehyd ist kommerziell erhältlich und kann beispielsweise durch Oxidation von Ethylenglykol hergestellt werden (siehe beispielsweise JP 3246248 und JP3279342). Glykolaldehyd wird bevorzugt durch Umsetzung von Formaldehyd mit Kohlenmonoxid und Wasserstoff synthetisiert, wie beispielsweise in der US2009012333, US2008081931, US2007249871, EP1697291, US 4503260 und US4322568 beschrieben.

Als weiterer Ausgangsstoff wird in das erfindungsgemäße Verfahren ein Aminiinierungsmittel eingesetzt.

Als Aminierungsmittel bei der hydrierenden Aminierung von Alkoholen, Aldehyden oder Ketonen in Gegenwart von Wasserstoff können sowohl Ammoniak als auch primäre oder sekundäre aliphatische oder cycloaliphatische oder aromatische Amine eingesetzt werden.

Das Aminierungsmittel ist bevorzugt eine Stickstoffverbindung der Formel I

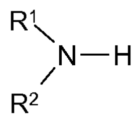

(I)

in der

R¹, R² Wasserstoff (H), Alkyl, wie $C_{1-20}$-Alkyl, Cycloalkyl, wie $C_{3-12}$-Cycloalkyl, Alkoxyalkyl, wie $C_{2-30}$-Alkoxyalkyl, Dialkylaminoalkyl, wie $C_{3-30}$-Dialkylaminoalkyl, Aryl, Aralkyl, wie $C_{7-20}$-Aralkyl, und Alkylaryl, wie $C_{7-20}$-Alkylaryl, oder gemeinsam $-(CH_2)_j-X-(CH_2)_k-$,

X $CH_2$, $CHR^3$ Sauerstoff (O), Schwefel (S) oder $NR^3$,

$R^3$ Wasserstoff (H), Alkyl, wie $C_{1-4}$-Alkyl, Alkylphenyl, wie $C_{7-40}$-Alkylphenyl,

j, k eine ganze Zahl von 1 bis 4,

bedeuten.

Besonders bevorzugt werden Ammoniak sowie die folgenden Mono- und Dialkylamine als Aminierungsmittel verwendet: Monomethylamin, Dimethylamin, Monoethylamin, Diethylamin, n-Propylamin, Di-n-propyl-amin, iso-Propylamin, Di-isopropyl-amin, I-sopropylethylamin, n-Butylamin, Di-n-Butylamin, s-Butylamin, Di-s-Butylamin, isoButylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin. Insbesondere bevorzugte Aminierungsmittel sind Ammoniak sowie Monomethylamin und Dimethylamin. Ganz besonders bevorzugt wird Ammoniak als Aminierungsmittel verwendet.

Als weiterer Einsatzstoff wird Wasserstoff in das erfindungsgemäße Verfahren eingesetzt.

Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Katalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Die Umsetzung von Glykolaldehyd mit einem Aminierungsmittel in Gegenwart von Wasserstoff findet erfindungsgemäß in einem Lösungsmittel statt.

Als Lösungsmittel kann jedes Lösungsmittel eingesetzt werden, dass sich unter den Reaktionsbedingungen inert verhält und eine ausreichende Löslichkeit für die Reaktionsedukte und Reaktionsprodukte aufweist.

Bevorzugte Lösungsmittel sind Wasser; Ether, wie Methyltertbutylether, Ethyltertbutylether, Dioxan oder Tetrahydrofuran (THF).

Als Lösungsmittel kommen auch geeignete Mischungen der zuvor aufgeführten Lösungsmittel in Betracht.

Besonders bevorzugte Lösungsmittel sind THF und Wasser.

Als besonders bevorzugte Lösungsmittel kommen auch die Reaktionsprodukte der erfindungsgemäßen Umsetzung von Glykolaldehyd und dem Aminierungsmittel in Betracht.

Das erfindungsgemäße Verfahren findet in Gegenwart eines Katalysators statt.

Die Katalysatoren können prinzipiell Nickel, Kobalt, Eisen, Kupfer, Chrom, Mangan, Kupfer, Molybdän, Wolfram und/oder andere der Metalle der Gruppen 8 und/oder 9 und/oder 10 und/oder 11 des Periodensystems der Elemente (Periodensystem in der IUPAC-Fassung vom 22.06.2007) enthalten.

Bevorzugt verwendet man Katalysatoren, die Kupfer, Kobalt und/oder Nickel enthalten.

Die oben genannten Katalysatoren können in üblicher Weise mit Promotoren, beispielsweise mit Chrom, Eisen, Kobalt, Mangan, Molybdän, Titan, Zinn, Metallen der Alkaligruppe, Metallen der Erdalkaligruppe und/oder Phosphor dotiert werden.

In einer bevorzugten Ausführungsform enthalten die Katalysatoren weniger als 25 Molprozent, bevorzugt weniger als 10 Molprozent, besonders bevorzugt weniger als 1 Molprozent, insbesondere bevorzugt weniger als 0,4 Molprozent und ganz besonders bevorzugt weniger als 0,1 Molprozent Edelmetallatome, bezogen auf die Gesamtzahl der Metallatome im Katalysator. Unter dem Begriff Edelmetalle werden im Rahmen der vorliegenden Erfindung Metalle ausgewählt aus der Gruppe bestehend aus Ruthenium, Rhodium, Palladium, Silber, Rhenium, Osmium, Iridium, Platin, Gold und Quecksilber bezeichnet.

Die im Katalysator vorhandene Anzahl von Metallatomen kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden.

In das erfindungsgemäße Verfahren werden Katalysatoren eingesetzt, die durch Reduktion von sogenannten Katalysatorvorläufern hergestellt werden.

Der Katalysatorvorläufer enthält eine aktive Masse, die eine oder mehrere katalytisch aktive Komponenten und optional ein Trägermaterial enthält.

Bei den katalytisch aktiven Komponenten handelt es sich um sauerstoffhaltige Verbindungen der oben genannten Metalle, beispielsweise um deren Metalloxide bzw. Hydroxide, wie CoO, NiO, CuO und/oder deren Mischoxide.

Im Rahmen dieser Anmeldung wird der Begriff katalytisch aktive Komponenten für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Die Katalysatorvorläufer können nach bekannten Verfahren, z.B. durch Fällung, Auffällung oder Imprägnierung hergestellt werden.

In einer bevorzugten Ausführungsform werden Katalysatorvorläufer in das erfindungsgemäße Verfahren eingesetzt, die durch Tränkung (Imprägnierung) von Trägermaterialien hergestellt werden (getränkte Katalysatorvorläufer).

Die Trägermaterialien, die bei der Imprägnierung verwendet werden, können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten.

Als Trägermaterialien kommen beispielsweise Kohlenstoff, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

In einer weiteren bevorzugten Ausführungsform werden Katalysatorvorläufer über eine gemeinsame Fällung (Mischfällung) aller ihrer Komponenten hergestellt. Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

Katalysatorvorläufer können weiterhin durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Nach der Konditionierung bzw. Formgebung erfolgt in der Regel eine Temperung. Die Temperaturen bei der Temperung entsprechen üblicherweise den Temperaturen bei der Calcinierung.

Die durch Fällungsreaktionen erhaltenen Katalysatorvorläufer enthalten die katalytisch aktiven Komponenten in Form eines Gemisches ihrer sauerstoffhaltigen Verbindungen, d.h. insbesondere als Oxide, Mischoxide und/oder Hydroxide. Die so hergestellten Katalysatorvorläufer können als solche gelagert werden.

Besonders bevorzugt sind Katalysatorvorläufer, wie

die in EP-A-0636409 offenbarten Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,2 bis 5,0 Gew.-% Alkali, berechnet als $M_2O$ (M=Alkali), enthalten, oder

in EP-A-0742045 offenbarte Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,05 bis 5 Gew.-% Alkali, berechnet als $M_2O$ (M=Alkali), enthalten, oder

in EP-A-696572 offenbarte Oxidgemische, die vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$, enthält, beispielsweise der in loc. cit, Seite 8, offenbarte Katalysator mit der Zusammensetzung 31,5 Gew.-% $ZrO_2$, 50 Gew.-% NiO, 17 Gew.-% CuO und 1,5 Gew.-% $MoO_3$, enthalten oder

in EP-A-963 975 offenbarte Oxidgemische, die vor der Reduktion mit Wasserstoff 22 bis 40 Gew.-% $ZrO_2$, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO, enthalten.

Der Katalysator, der in das erfindungsgemäße Verfahren eingesetzt wird, wird dadurch erhalten, dass man Katalysatorvorläufer, die wie voranstehend beschrieben durch Imprägnierung oder Fällung hergestellt wurden, nach der Calcinierung bzw. Konditionierung reduziert.

Die Reduktion des trockenen, in der Regel pulverförmigen Katalysatorvorläufers kann bei erhöhter Temperatur in einem bewegten oder unbewegten Reduktionsofen durchgeführt werden.

Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit Frisch-Wasserstoff und ggf. nach Entfernen von Wasser durch Kondensation.

Die Reduktion des Katalysatorvorläufers erfolgt bevorzugt in einem Reaktor, in dem die Katalysatorformkörper als Festbett angeordnet sind. Besonders bevorzugt erfolgt die Reduktion des Katalysatorvorläufers in demselben Reaktor in dem die nachfolgende Umsetzung von Glykolaldehyd mit Aminierungsmittel erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Die Reduktion des Katalysatorvorläufers kann auch in Suspension erfolgen, beispielsweise in einem Rührautoklaven. Die Temperaturen liegen im Allgemeinen in einem Bereich von 50 bis 300°C, insbesondere von 100 bis 250°C, besonders bevorzugt von 120 bis 200°C.

Die Reduktion in Suspension wird in der Regel bei einem Wasserstoffpartialdruck von 1 bis 300 bar, bevorzugt von 10 bis 250 bar, besonders bevorzugt von 30 bis 200 bar durchgeführt. Als Lösungsmittel kommen die voranstehend genannten Lösungsmittel in Betracht.

Die Dauer der Reduktion in Suspension beträgt bevorzugt 5 bis 20 Stunden, besonders bevorzugt 8 bis 15 Stunden.

Der Katalysator kann nach der Reduktion unter inerten Bedingungen gehandhabt werden. Bevorzugt kann der Katalysator unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Katalysators.

Der Katalysator kann nach der Reduktion aber auch mit einem Sauerstoff enthaltenden Gasstrom wie Luft oder einem Gemisch von Luft mit Stickstoff in Kontakt gebracht. Dadurch wird ein passivierter Katalysator erhalten. Der passivierte Katalysator weist im Allgemeinen eine schützende Oxidschicht auf. Durch diese schützende Oxidschicht wird die Handhabung und Lagerung des Katalysatorsvereinfacht, so dass beispielsweise der Einbau des passivierten Katalysators in den Reaktor vereinfacht wird.

Erfindungsgemäß wird Glykolaldehyd mit einem aktivierten Katalysator in Kontakt gebracht.

Erfindungsgemäß wird ein Katalysator durch Reduktion eines Katalysatorvorläufers oder durch Reduktion eines passivierten Katalysators aktiviert.

Im Rahmen der vorliegenden Erfindung ist ein aktivierter Katalysator ein Katalysator, der durch Reduktion eines Katalysatorvorläufers hergestellt wurde und der während und nach der Reduktion bis zum Inkontaktbringen mit Glykolaldehyd unter inerten Bedingungen gehandhabt wurde.

Im Rahmen der vorliegenden Erfindung ist ein aktivierter Katalysator auch ein Katalysator, der durch Reduktion eines passivierten Katalysators hergestellt wurde und der während und nach der Reduktion bis zum Inkontaktbringen mit Glykolaldehyd unter inerten Bedingungen gehandhabt wurde

In einem solchen Katalysator liegen die Metalle zum Teil in reduzierter Form vor und ein solcher Katalysator weißt im Allgemeinen keine schützenden Oxidschicht auf.

Ein Maß für die Aktivierung eines Katalysators ist der Reduktionsgrad.

In einer bevorzugten Ausführungsform beträgt der Reduktionsgrad des aktivierten Katalysators 30% und mehr, bevorzugt 50% und mehr, besonders bevorzugt 75% und mehr und insbesondere bevorzugt 90% und mehr.

In einer bevorzugten Ausführungsform weist ein aktivierter Katalysator, der durch Reduktion eines passivierten Katalysators hergestellt wurde, nach der Aktivierung einen Reduktionsgrad auf, der mindestens 2%, bevorzugt mindestens 3% und besonders bevorzugt mindestens 4% über dem Reduktionsgrad des passivierten Katalysators liegt.

Die Bestimmung des Reduktionsgrad erfolgt im Allgemeinen durch "Temperaturprogrammierte Reduktion" (TPR).

Die temperaturprogrammierte Reduktion erfolgt durch Aufheizen der Probe des Katalysatorvorläufers in einem Wasserstoff/Inertgas-Strom mit konstanter Temperaturerhöhung pro Zeiteinheit. Man verwendet bevorzugt eine Anordnung, deren Aufbau auf den Vorschlägen von Monti und Baiker [D. A. M. Monti, A. Baiker, "Temperatur-Programmed Reduction. Parametric Sensitivity and Estimation of Kinetic Parameters", J. Catal. 83 (1983) 323-335] beruht.

In dieser Messanordnung werden die pulverförmigen Proben als lose Schüttung zwischen zwei Glaswollepropfen in ein U-förmiges Glasrohr eingefüllt. Das U-Rohr befindet sich in einem Röhrenofen aus Keramik. Die Probe wird nach Einbau in die TPR-Apparatur zunächst getrocknet, indem sie in einem Argonstrom bis 200 °C aufgeheizt und dort 30 Minuten gehalten wird. Anschließend wird auf 50 °C abgekühlt. Die Probe wird mit einer Heizrampe von 5 K/min von 50 °C auf eine Endtemperatur von 650 °C aufgeheizt. Die Probentemperatur wird in einer Thermoelementhülse nahe der Schüttung gemessen und in Intervallen von 2 s aufgezeichnet. Durch das U-Rohr wird ein Wasserstoff/Argon-Strom mit 10 % Wasserstoff geleitet. Der Wasserstoffgehalt im Abgas wird mit einem Wärmeleitfähigkeitsdetektor bestimmt. Der Wasserstoffverbrauch wird in Abhängigkeit von der Temperatur aufgezeichnet. Durch Integration wird der gesamte $H_2$-Verbrauch im untersuchten Temperaturintervall ermittelt.

Aus dem $H_2$-Verbrauch kann der Reduktionsgrad RG nach folgender Formel berechnet werden:

RG = 100 % - 100 % * [(Gemessener Wasserstoff-Verbrauch der Katalysatorprobe (aus TPR-Messung)) / (theoretischer Wasserstoff-Verbrauch des volloxidischen Katalysators, welcher aufgrund der Metallgehalte der Probe und Reaktionsstöchiometrie berechnet wird)]

Bei der Berechnung des theoretischen Wasserstoff-Verbrauchs wird die Annahme gemacht, dass Ni, Cu und Co als NiO, CuO und CoO vorliegen und die vorgenannten Promotoren nicht reduziert vorliegen. Bei der Berechnung des Reduktionsgrad werden nämlich üblicherweise nur solche Metalloxide betrachtet, die unter den Bedingungen der TPR-Messung zu den korrespondierenden Metallen reduziert werden. Beispielsweise wird $ZrO_2$ unter den Bedingungen der TPR-Messung nicht reduziert, so dass der Zr-Gehalt bei der Bestimmung des Reduktionsgrades nicht berücksichtigt wird.

Die Aktivierung des Katalysators erfolgt durch Reduktion eines Katalysatorvorläufers. Die Reduktion eines Katalysatorvorläufers wurde bereits voranstehend beschrieben.

Die Aktivierung eines Katalysators kann auch durch Reduktions eines passivierten Katalysators erfolgen. Die Reduktion eines passivierten Katalysators kann, wie voranstehend beschrieben durch Behandlung des passivierten Katalysators mit Wasserstoff oder einem Wasserstoff enthaltenden Gas erfolgen. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

Ein aktivierter Katalysator muss während und nach seiner aktivierenden Reduktion unter inerten Bedingungen gehandhabt werden.

Bevorzugt wird der aktivierte Katalysator unter einem Inertgas, wie Stickstoff, oder unter einer inerten Flüssigkeit, zum Beispiel einem Alkohol, Wasser oder dem Produkt der jeweiligen Reaktion, für die der Katalysator eingesetzt wird, gehandhabt und gelagert. Gegebenenfalls muss der aktivierte Katalysator vor Beginn der eigentlichen Reaktion dann von der inerten Flüssigkeit befreit werden.

Erfindungsgemäß wird das Glykolaldehyd mit dem aktivierten Katalysator in Kontakt gebracht.

Erfindungsgemäß wird der aktivierte Katalysator bis zum Inkontaktbringen während und nach der Aktivierung unter inerten Bedingungen gehandhabt. Vorzugsweise erfolgt auch das Inkontaktbringen des Glykolaldehyds mit dem

aktivierten Katalysator unter inerten Bedingungen, besonders bevorzugt in Gegenwart von Wasserstoff oder einem Wasserstoff enthaltenden Gas.

In einer bevorzugten Ausführungsform wird der aktivierte Katalysator mit Glykolaldehyd in dem Reaktor in Kontakt gebracht, in dem zuvor bereits die Aktivierung des Katalysators erfolgt ist. Erfindungsgemäß wird der aktivierte Katalysator bis zum Inkontaktbringen während und nach der Aktivierung unter inerten Bedingungen gehandhabt, vorzugsweise in Gegenwart von Wasserstoff oder einem Wasserstoff enthaltenden Gas. Alternativ kann der aktivierte Katalysator nach seiner Aktivierung in Gegenwart von Stickstoff oder einem anderen geeigneten Inertgas gelagert werden. Hierzu wird in der Regel der Anteil des Inertgases im Wasserstoffstrom nach der Aktivierung sukzessiv erhöht. Vorzugsweise erfolgt auch die Dosierung des Glykolaldehyds unter inerten Bedingungen, bevorzugt in Gegenwart von Wasserstoff oder eines Inertgases.

In einer weiteren bevorzugten Ausführungsform wird der aktivierte Katalysator nach der Aktivierung mit einer inerten Flüssigkeit in Kontakt gebracht.

Bevorzugt erfolgt das Inkontaktbringen des aktivierten Katalysators mit einer inerten Flüssigkeit dadurch, dass die inerte Flüssigkeit zu dem aktivierten Katalysator zudosiert wird. Vorzugsweise findet die erfindungsgemäße Umsetzung von Glykolaldehyd in demselben Reaktor statt, in dem auch die Aktivierung des Katalysators vorgenommen wurde.

Der Katalysator kann aber auch zusammen mit der inerten Flüssigkeit in den Reaktor überführt werden, in dem das Inkontaktbringen mit Glykolaldehyd erfolgt. Das Glykolaldehyd kann bereits als Vorlage im Reaktor vorhanden sein, es kann aber auch nach der Überführung des Katalysators in den Reaktor zudosiert werden. Vorzugsweise findet das Inkontaktbringen des aktivierten Katalysators mit Glykolaldehyd unter inerten Bedingungen statt, besonders bevorzugt in Gegenwart von Wasserstoff oder eines Inertgases.

In dem erfindungsgemäßen Verfahren wird Glykolaldehyd mit einem Aminierungsmittel in Gegenwart von Wasserstoff und einem Lösungsmittel umgesetzt.

Das Lösungsmittel kann in einem Anteil von 5 bis 95 % Gew.-%, bevorzugt 20 bis 70 %, besonders bevorzugt 30 bis 60 %, eingesetzt werden, jeweils bezogen auf das Gesamtgewicht des Reaktionsgemisches, wobei das Gesamtgewicht des Reaktionsgemisches aus der Summe der Massen der in das Verfahren eingesetzten Ausgangsstoffe (Glykolaldehyd und Aminierungsmittel) und Lösungsmittel ist.

Das Verhältnis von Aminierungsmittel zu eingesetztem Glykolaldehyd liegt üblicherweise in einem Bereich von 1:100 bis 100:1, bevorzugt 1:1 bis 50:1 und besonders bevorzugt 1:1 bis 45:1

Die Reaktion wird üblicherweise bei einem Druck von 1 bis 500 bar, bevorzugt 10 bis 350 bar, besonders bevorzugt bei einem Druck von 50 bis 300 bar und ganz besonders bevorzugt 80 bis 220 bar durchgeführt. Die Druckhaltung bzw. Drucksteuerung erfolgt in der Regel über die Dosierung des Wasserstoffs.

Die Umsetzung von Glykolaldehyd mit Aminierungsmittel erfolgt im Allgemeinen bei Temperaturen von 15 bis 350°C, bevorzugt 50 bis 250°C, besonders bevorzugt 80 bis 220°C.

In einer besonders bevorzugten Ausführungsform beträgt das Verhältnis von Aminierungsmittel zu eingesetztem Glykolaldehyd bevorzugt 1:100 bis 100:1, besonders bevorzugt 1:1 bis 50:1 und ganz besonders bevorzugt 1:1 bis 45:1. In dieser besonders bevorzugten Ausführungsform beträgt der Druck bevorzugt 1 bis 200 bar, besonders bevorzugt 10 bis 150 bar und ganz besonders bevorzugt 50 bis 120 bar und die Temperatur beträgt bevorzugt 20 bis 300°C, besonders bevorzugt 50 bis 250°C und ganz besonders 80 bis 120 °C. In dieser besonderen Ausführungsform wird bei der Umsetzung von Glykolaldehyd im Allgemeinen MEOA mit hoher Selektivität und Ausbeute gebildet.

In einer weiteren besonders bevorzugten Ausführungsform beträgt das Verhältnis von Aminierungsmittel zu eingesetztem Glykolaldehyd bevorzugt 1:100 bis 100:1, besonders bevorzugt 1:1 bis 50:1 und ganz besonders bevorzugt 1:1 bis 45:1. In dieser besonders bevorzugten Ausführungsform beträgt der Druck bevorzugt 100 bis 300 bar, besonders bevorzugt 150 bis 250 bar und ganz besonders bevorzugt 180 bis 220 bar und die Temperatur beträgt bevorzugt 20 bis 300°C, besonders bevorzugt 50 bis 250°C und ganz besonders 160 bis 220 °C. In dieser besonderen Ausführungsform wird bei der Umsetzung von Glykolaldehyd im Allgemeinen EDA mit hoher Selektivität und Ausbeute gebildet.

Das erfindungsgemäße Verfahren kann kontinuierlich, diskontinuierlich oder semikontinuierlich durchgeführt werden.

Typische Reaktoren sind beispielsweise Hochdruck-Rührkessel-Reaktoren, Autoklaven, Festbettreaktoren, Wirbelschichtreaktoren, Wanderbetten, zirkulierende Wirbelschichten, Salzbadreaktoren, Plattenwärmetauscher als Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, kontinuierlich gerührte Kessel, Blasenreaktoren usw., wobei jeweils der für die gewünschten Reaktionsbedingungen (wie Temperatur, Druck und Verweilzeit) geeignete Reaktor eingesetzt wird.

Bevorzugt wird das erfindungsgemäße Verfahren in einem Hochdruck-Rührkessel-Reaktor, Festbettreaktor oder Wirbelschichtreaktor durchgeführt.

In einer besonders bevorzugten Ausführungsform wird das erfindungsgemäße Verfahren in ein oder mehreren Festbettreaktoren durchgeführt.

In einer weiteren besonders bevorzugten Ausführungsform erfolgt die Umsetzung von Glykolaldehyd in einem

Hochdruck-Rührkessel-Reaktor.

Das Glykolaldehyd und das Aminierungsmittel können gemeinsam in die Reaktionszone des Reaktors gegeben werden, zum Beispiel als vorgemischter Reaktandenstrom, oder getrennt. Bei einer getrennten Zugabe können das Glykolaldehyd und das Aminierungsmittel entweder gleichzeitig, zeitversetzt oder nacheinander in die Reaktionszone des Reaktors gegeben werden.

Die Verweilzeit beträgt in dem erfindungsgemäßen Verfahren bei der Durchführung in einem diskontinuierlichen Verfahren im Allgemeinen 15 Minuten bis 72 Stunden, bevorzugt 60 Minuten bis 24 Stunden, besonders bevorzugt 2 Stunden bis 10 Stunden.

Bei Durchführung in einem bevorzugten kontinuierlichen Verfahren beträgt die Katalysatorbelastung im Allgemeinen im Bereich von 0,01 kg Glykolaldhyd / kg Katalysator/h bis 3,0 kg Glykolaldhyd / kg Katalysator /h, bevorzugt 0,05 kg Glykolaldhyd / kg Katalysator /h bis 2.0 kg Glykolaldhyd / kg Katalysator /h und besonders bevorzugt 0,1 kg Glykolaldhyd / kg Katalysator /h - 1,5 kg Glykolaldhyd / kg Katalysator /h.

Im Anschluss an die erfindungsgemäße Umsetzung kann die Isolierung des gewünschten Produktes nach dem Fachmann bekannten Verfahren erfolgen, zum Beispiel durch Destillation.

Die Vorteile der vorliegenden Erfindung bestehen darin, dass ein Verfahren zur Umsetzung von Glykolaldehyd entwickelt werden konnte, dass einen hohen Umsatz von Glykolaldehyd und die Bildung der Produkte, insbesondere von MEOA und/oder EDA, in hoher Ausbeute und Selektivität ermöglicht. Weiterhin wird die Bildung des unerwünschten Nebenprodukts Piperazin verringert. Zudem fallen die Umsetzungsprodukte in einer hohen Reinheit an. Diese Ziele wurden unter der Prämisse erreicht, dass in das erfindungsgemäße Verfahren Katalysatoren eingesetzt werden können, die weitestgehend frei von Edelmetallen ist. Somit können die Stoffkosten des Verfahrens gesenkt werden. Die Verwendung von edelmetallhaltigen Katalysatoren führt nämlich zu einer starken Erhöhung der Katalysatoreinsatzkosten, die sich auf die Wirtschaftlichkeit des Verfahrens auswirken. In Zukunft ist mit einer drastischen Verknappung von Rohstoffen zu rechnen, so dass erwartet werden kann, dass die Preise für Edelmetalle noch weiter ansteigen.

Weiterhin weisen die Katalysatoren in dem erfindungsgemäßen Verfahren eine hohe mechanische und chemische Beständigkeit auf, so dass hohe Standzeiten erreicht werden können.

Das erfindungsgemäße Verfahren wird anhand der nachfolgend aufgeführten Beispiele näher ausgeführt.

Herstellung der Katalysatorvorläufer:

Katalysatorvorläufer a)

Eine wässrige Lösung aus Nickelnitrat, Kupfernitrat und Zirconacetat, die 4,48 Gew.-% Ni (berechnet als NiO), 1,52 Gew.-% Cu (berechnet als CuO und 2,82 Gew.-% Zr (berechnet als $ZrO_2$) enthält, wird gleichzeitig in einem Rührgefäß in einem konstanten Strom mit einer 20 % igen wässrigen Natriumcarbonatlösung bei einer Temperatur von 70°C so gefällt, dass der mit einer Glaselektrode gemessene pH-Wert von 7,0 aufrechterhalten wird. Die erhaltene Suspension wird filtriert und der Filterkuchen mit voll entsalztem Wasser gewaschen bis die elektrische Leitfähigkeit des Filtrats ca. 20 $\mu$S beträgt. Dann wird in den noch feuchten Filterkuchen so viel Ammoniumheptamolybdat eingearbeitet, dass das nachfolgend angegebene Oxidgemisch erhalten wird. Danach wird der Filterkuchen bei einer Temperatur von 150°C in einem Trockenschrank oder einem Sprühtrockner getrocknet. Das auf diese Weise erhaltene Hydroxid-Carbonat-Gemisch wird nun bei einer Temperatur von 430 bis 460°C über einen Zeitraum von 4 Stunden getempert. Die so hergestellte Katalysatorvorläufer hat die Zusammensetzung: 50 Gew.-% NiO, 17 Gew.-% CuO, 1,5 Gew.-% $MoO_3$ und 31,5 Gew.-% $ZrO_2$. Der Katalysator wurde mit 3 Gew.-% Graphit vermischt und zu Tabletten verformt.

Herstellung von Katalysatorvorläufer (b):

Eine wässrige Lösung aus Nickelnitrat, Kobaltnitrat, Kupfernitrat und Zirkoniumacetat die 2,39 Gew.-% NiO, 2,39 Gew.-% CoO, 0,94 Gew.-% CuO und 2,82 Gew.-% $ZrO_2$ enthielt, wurde gleichzeitig in einem Rührgefäß in einem konstanten Strom mit einer 20 % igen wässrigen Natriumcarbonatlösung bei einer Temperatur von 70°C so gefällt, dass der mit einer Glaselektrode gemessene pH-Wert von 7,0 aufrechterhalten wurde. Die erhaltene Suspension wurde filtriert und der Filterkuchen mit voll entsalztem Wasser gewaschen bis die elektrische Leitfähigkeit des Filtrats ca. 20 $\mu$S betrug. Danach wurde der Filterkuchen bei einer Temperatur von 150°C in einem Trockenschrank oder einem Sprühtrockner getrocknet. Das auf diese Weise erhaltene Hydroxid-Carbonat-Gemisch wurde nun bei einer Temperatur von 450 bis 500 °C über einen Zeitraum von 4 Stunden getempert. Der so hergestellte Katalysatorvorläufer hatte die Zusammensetzung: 28 Gew.-% NiO, 28 Gew.-% CoO, 11 Gew.-% CuO und 33 Gew.-% $ZrO_2$. Der Katalysatorvolräufer wurde mit 3 Gew.-% Graphit vermischt und zu Tabletten verformt.

Herstellung von Katalysatorvorläufer (c):

Durch Auflösen von Kobaltnitrat, Mangannitrat und Phosphorsäure in Wasser wurde eine Lösung hergestellt, die 10 Gew.-% Kobalt, 0,55 Gew.-% Mangan und 0,45 Gew.-% $H_3PO_4$ enthält. Durch Zugabe einer 20%igen Natriumcarbonatlösung wurde bei einer Temperatur von 50°C gefällt. Der entstandene Niederschlag wurde gewaschen, bis im Waschwasser kein Natrium oder Nitrat mehr nachweisbar war. Der so erhaltene Feststoff wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser verformt. Die Stränge wurden bei 100 bis 120°C getrocknet und anschließend 1 h bei 650°C und danach 3 h bei 850°C calziniert.
Der so hergestellte Katalysatorvorläufer enthielt 90,4 Gew.-% Kobalt, 5,1 Gew.-% Mangan, 0,3 Gew.-% Natrium und 3,1 Gew.-% Phosphor.

Herstellung von Katalysatorvorläufer (d):

Die Herstellung des Katalysatorvorläufers (d) erfolgte gemäß Beispiel 1A der EP-A-1317959, jedoch ohne Verwendung von Eisen (III)-chlorid.

Herstellung von Katalysator (e):

Als Katalysator (e) wurde kommerziell erhältliches Ni-1404 der Fa. Engelhardt (Engelhardt Co., Iselin, New Jersey) eingesetzt. Dieser Katalysator besteht aus Ni und NiO sowie Aluminium- und Siliziumoxiden. Der Katalysator wurde bereits vom Hersteller in reduzierter und passivierter Form geliefert und wurde pulverförmig eingesetzt.

Reduktion und Passivierung der Katalysatorvorläufer

Die oxidischen Tabletten (Katalysatorvorläufer (a) und (b)) bzw. Stränge (Katalysatorvorläufer (c)) oder Pulver (Katalysatorvorläufer (d)) wurden reduziert. Die Reduktion wurde je nach Katalysatorvorläufer im Bereich von 250 bis 500°C°C durchgeführt, wobei die Aufheizungsrate 3°C/Minute betrug. Zuerst wurde 50 Minuten mit 10 % $H_2$ in $N_2$ reduziert, anschließend 20 Minuten mit 25 % $H_2$ in $N_2$, dann 10 Minuten mit 50 % $H_2$ in $N_2$, dann 10 Minuten mit 75 % $H_2$ in $N_2$ und schließlich 3 Stunden mit 100 % $H_2$. Bei den %-Angaben handelt es sich jeweils um Volumen-%. Die Passivierung der reduzierten Katalysatoren wurde bei Raumtemperatur in verdünnter Luft (Luft in $N_2$ mit einem $O_2$-Gehalt von maximal 5 Vol.-%) durchgeführt.

Umsetzungen von Glykolaldehyd:

Beispiele 1 bis 12:

In einem elektrobeheizten 160 ml-Autoklaven (Hastelloy) mit mechanischem, magnetgekuppeltem Rührer wurden 3 g käuflicher, dimerer Glykolaldehyd (50 mmol, berechnet als Monomer) im jeweiligen Lösungsmittel vorgelegt (20 ml). Anschließend wurde unter Inertgasatmosphäre die in Tabelle 1 angegebene Menge des aktivierten Katalysators, suspendiert in 10 ml THF zugegeben.
Der passivierte Katalysator wurde vor Einbringen in den Autoklaven wie folgt aktiviert:
In den Beispielen 1 bis 3 wurde der passivierte Katalysator 10 Stunden bei 280°C bei einem Partialwasserstoffdruck von 1 bar reduziert.
Der Reduktionsgrad betrug in allen Fällen mehr als 30 %.
In den Beispielen 4 bis 11 wurde der passivierte Katalysator 10 Stunden bei 280°C bei einem Partialwasserstoffdruck von 1 bar reduziert.
Der Reduktionsgrad betrug in allen Fällen mehr als 30%.
In Beispiel 12 wurde der passivierte Katalysator nicht aktiviert (Vergleichsbeispiel).
Anschließend wurde Ammoniak, entsprechend dem in Tabelle 1 angegebenen Molverhältnis (Ammoniak:monomerer Glykolaldehyd), zudosiert und die Mischung auf 100 °C erwärmt. Bei Erreichen dieser Temperatur wurde soviel Wasserstoff aufgedrückt, dass der angegebene Reaktionsdruck erreicht wurde. Während der Reaktion wurde der Druck durch weitere Zufuhr von Wasserstoff aufrecht erhalten und der Verbrauch gemessen. In allen Fällen wurde 8 h bei 100 °C und dem jeweiligen Druck gerührt. Der Umsatz wurde mit Hilfe des Wasserstoffverbrauchs näherungsweise bestimmt.
Der Reaktionsaustrag nach 8 h wurde vom Katalysator abfiltriert, mit Methanol versetzt und per GC (Flächenprozente) analysiert.
Die Differenz zu 100 % sind nicht-identifizierte Nebenkomponenten.

Beispiele 13 bis 16:

In einem elektrobeheizten 160 ml-Autoklaven (Hastelloy) mit mechanischem, magnetgekuppeltem Rührer wurden 3 g käuflicher, dimerer Glykolaldeyd (50 mmol, berechnet als Monomer) in 20 ml THF vorgelegt. Es wurden 500 µl (0.47 g) Diethylenglykoldimethylether als interner Standard zugegeben Anschließend wurde unter Inertgasatmosphäre 0.5 g Katalysator, suspendiert in 10 ml THF zugegeben.

Im Falle der Beispiele 14 und 16 wurde der passivierte Katalysator jeweils vor der Zugabe in den Reaktor mit Wasserstoff 10 Stunden lang bei 250 °C bei einem Wasserstoffpartialdruck von 1 bar aktiviert.

Der Reduktionsgrad ist in Tabelle 2 angegeben.

Bei Beispielen 13 und 15 wurde der passivierte Katalysator vor der Zugabe in den Reaktor nicht aktiviert.

Anschließend wurden 30 g Ammoniak, entsprechend einem Molverhältnis (Ammoniak:monomerer Glykolaldehyd) von 35:1, zudosiert und die Mischung auf 100 °C erwärmt. Bei Erreichen dieser Temperatur wurde soviel Wasserstoff aufgedrückt, dass ein Reaktionsdruck von 80 bar erreicht wurde. Während der Reaktion wurde der Druck durch weitere Zufuhr von Wasserstoff aufrecht erhalten und der Verbrauch gemessen. Die Reaktion wurde bei 100 °C und 800 Upm gerührt. Die Reaktionsdauer ist in der Tabelle 2 angegeben. Der Reaktionsaustrag wurde vom Katalysator abfiltriert, mit Methanol versetzt und per GC (Flächenprozente) analysiert. Ferner wurde mit Hilfe des internen Standards die Ausbeute an den aufgeführten Verbindungen bestimmt (Tabelle 2).

Bestimmung des Reduktionsgrads:

Die Messung wurde auf Gerät Micromeritics RS 232, Autochem II Chemisorption analyser aufgenommen. Als Auswertungssoftware wurde das Programm Autochem II 2920 verwendet.

Die temperaturprogrammierte Reduktion erfolgte durch Aufheizen der Probe des Katalysatorvorläufers in einem Wasserstoff/Inertgas-Strom mit konstanter Temperaturerhöhung pro Zeiteinheit. Es wurde eine Anordnung verwendet, deren Aufbau auf den Vorschlägen von Monti und Baiker [D. A. M. Monti, A. Baiker, "Temperatur-Programmed Reduction. Parametric Sensitivity and Estimation of Kinetic Parameters", J. Catal. 83 (1983) 323-335] beruht. Die pulverförmigen Proben wurden als lose Schüttung zwischen zwei Glaswollepropfen in ein U-förmiges Glasrohr eingefüllt. Das U-Rohr befindet sich in einem Röhrenofen aus Keramik. Die Probe wurde nach Einbau in die TPR-Apparatur zunächst getrocknet, indem sie in einem Argonstrom bis 200 °C aufgeheizt und dort 30 Minuten gehalten wurde. Anschließend wurde auf 50 °C abgekühlt. Die Probe wurde mit einer Heizrampe von 5 K/min von 50 °C auf eine Endtemperatur von 650 °C aufgeheizt. Die Probentemperatur wurde in einer Thermoelementhülse nahe der Schüttung gemessen und in Intervallen von 2 s aufgezeichnet. Durch das U-Rohr wurde ein Wasserstoff/Argon-Strom mit 10 % Wasserstoff geleitet. Der Wasserstoffgehalt im Abgas wurde mit einem Wärmeleitfähigkeitsdetektor bestimmt. Der Wasserstoffverbrauch wurde in Abhängigkeit von der Temperatur aufgezeichnet. Durch Integration wurde der gesamte $H_2$-Verbrauch im untersuchten Temperaturintervall ermittelt. Aus dem $H_2$-Verbrauch wurde der Reduktionsgrad RG nach folgender Formel berechnet:

RG = 100 % - 100 % * [(Gemessener Wasserstoff-Verbrauch der Katalysatorprobe (aus TPR-Messung)) / (theoretischer Wasserstoff-Verbrauch des volloxidischen Katalysators, welcher aufgrund der Metallgehalte der Probe und Reaktionsstöchiometrie berechnet wird)]

Tabelle 1. Aminierung von Glykolaldehyd mit $NH_3$ zu MEOA.

| Beispiel | Verwendeter Katalysatorvorläufer | Kat.menge [g] | Solvens | Temperatur [°C] | Druck [bar] | Molverhältnis NH3:GA (monomer) | Umsatz [%] | EDA [%] | MEOA [%] | Piperazin [%] | AEEA [%] | DEOA [%] | TEOA [%] |
|---|---|---|---|---|---|---|---|---|---|---|---|---|---|
| | | | | | | | | | | | | | |
| 1 | (a) | 0,5 | THF | 100 | 100 | 35 | 100 | 17,2 | 68,7 | 2,3 | 4,6 | 0,0 | 0,0 |
| 2 | (a) | 0,5 | THF | 100 | 100 | 50 | 100 | 13,5 | 76,7 | 1,1 | 3,2 | 0,3 | 0,0 |
| 3 | (b) | 0,5 | THF | 100 | 100 | 35 | 100 | 6,0 | 60,7 | 0,6 | 0,0 | 1,5 | 0,0 |
| 4 | (c) | 0,5 | THF | 100 | 100 | 35 | 100 | 12,7 | 76,5 | 1,3 | 3,4 | 0,3 | 0,0 |
| 5 | (c) | 0,5 | THF | 100 | 60 | 35 | 90 | 12,63 | 67,4 | 2,1 | 3,2 | 1,3 | 0,0 |
| 6 | (c) | 0,5 | THF | 100 | 80 | 35 | 100 | 6,35 | 82,6 | 0,6 | 2,3 | 1,9 | 0,4 |
| 7 | (c) | 0,5 | THF | 100 | 100 | 35 | 100 | 5,77 | 75,1 | 0,5 | 2,2 | 2,5 | 0,4 |
| 8 | (c) | 0,5 | THF | 100 | 100 | 10 | 90 | 5,43 | 68,6 | 0,8 | 3,1 | 9,5 | 0,4 |
| 9 | (c) | 0,5 | THF | 100 | 40 | 10 | 100 | 12,33 | 61,6 | 3,2 | 4,3 | 9,5 | 0,0 |
| 10 | (c) | 0,5 | THF | 100 | 100 | 60 | 100 | 6,83 | 73,5 | 0,6 | 1,5 | 3,8 | 0,0 |
| 11 | (c) | 0,5 | Wasser | 100 | 100 | 35 | > 80 | 0,4 | 71,3 | n.b. | n.b. | 1,5 | 0,0 |
| 12 | (d) | 0,23 | THF | 100 | 100 | 35 | 60 | 23,20 | 59,7 | 7,5 | 2,6 | 0,0 | 0,0 |

Tabelle 2. Aminierung von Glykolaldehyd mit $NH_3$ an reduzierten/passivierten Katalysatoren und an aktivierten Katalysatoren.

| Beispiel | Verwendeter Katalysatorvorläufer | Katalysator-Vorbehandlung | Reduktionsgrad nach TPR [%] | Reaktionsdauer [h] | EDA [%] | MEOA [%] | Piperazin [%] | AEEA [%] | DEOA [%] | TEOA [%] | Ausbeute EDA [%] | Ausbeute MEOA [%] | Ausbeute Piperazin [%] |
|---|---|---|---|---|---|---|---|---|---|---|---|---|---|
| | | | | | | | | | | | | | |
| 13 | (a) | keine | 24 | 5,1 | 20,6 | 56,9 | 3,98 | 4,64 | 0,34 | 0,0 | 14,23 | 44,37 | 1,36 |
| 14 | (a) | Aktivierung mit H2 | 50 | 1,2 | 7,8 | 82,1 | 0,60 | 2,71 | 0,68 | 0,0 | 6,13 | 72,74 | 0,23 |
| 15 | (e) | keine | 51 | 2,4 | 14,2 | 60,0 | 2,90 | 3,78 | 0,57 | 0,0 | 7,27 | 34,04 | 0,13 |
| 16 | (e) | Aktivierung mit H2 | 55 | 1,2 | 4,6 | 85,2 | 0,32 | 1,83 | 2,01 | 0,0 | 3,46 | 72,51 | 0,12 |

## Patentansprüche

1. Verfahren zur Umsetzung von Glykolaldehyd mit einem Aminierungsmittel in Gegenwart von Wasserstoff und eines Katalysators, wobei man den Katalysator durch Reduktion eines Katalysatorvorläufers oder durch Reduktion eines passivierten Katalysators aktiviert, **dadurch gekennzeichnet, dass** die Umsetzung in Gegenwart eines Lösungsmittels erfolgt und dass der Glykolaldehyd mit dem aktivierten Katalysator in Kontakt gebracht wird.

2. Verfahren nach Anspruch 1, **dadurch gekennzeichnet, dass** der Katalysatorvorläufer als katalytisch aktive Komponenten ein oder mehrere sauerstoffhaltige Verbindungen der Metalle der Gruppen 8 und/oder 9 und/oder 10 und/oder 11 des Periodensystems der Elemente enthält.

3. Verfahren nach mindestens einem der Ansprüche 1 bis 2, **dadurch gekennzeichnet, dass** die katalytische aktive Komponente eine sauerstoffhaltige Verbindung von Ni, Co und/oder Cu ist.

4. Verfahren nach mindestens einem der Ansprüche 1 bis 3, **dadurch gekennzeichnet, dass** der eingesetzte Katalysator weniger als 0,4 Molprozent Edelmetallatome ausgewählt aus der Gruppe bestehend aus Ruthenium, Rhodium, Palladium, Silber, Rhenium, Osmium, Iridium, Platin, Gold und Quecksilber enthält.

5. Verfahren nach mindestens einem der Ansprüche 1 bis 4, **dadurch gekennzeichnet, dass** man die Umsetzung bei einer Temperatur von 15 bis 350°C durchführt.

6. Verfahren nach mindestens einem der Ansprüche 1 bis 5, **dadurch gekennzeichnet, dass** man die Umsetzung bei einem Druck von 10 bis 350 bar durchführt.

7. Verfahren nach mindestens einem der Ansprüche 1 bis 6, **dadurch gekennzeichnet, dass** das Lösungsmittel THF oder Wasser ist.

8. Verfahren nach mindestens einem der Ansprüche 1 bis 7, **dadurch gekennzeichnet, dass** der aktivierte Katalysator einen Reduktionsgrad von 30% und mehr aufweist.

9. Verfahren nach mindestens einem der Ansprüche 1 bis 8, **dadurch gekennzeichnet, dass** der aktivierte Katalysator, der durch Reduktion eines passivierten Katalysators hergestellt wurde, nach der Aktivierung einen Reduktionsgrad aufweist, der mindestens 2% über dem Reduktionsgrad des passivierten Katalysators liegt.

10. Verfahren nach mindestens einem der Ansprüche 1 bis 9, **dadurch gekennzeichnet, dass** der aktivierte Katalysator während und nach der Reduktion bis zum Inkontaktbringen mit Glykolaldehyd unter inerten Bedingungen gehandhabt wird.

## Claims

1. A process for reacting glycolaldehyde with an aminating agent in the presence of hydrogen and of a catalyst, the catalyst being activated by reducing a catalyst precursor or by reducing a passivated catalyst, which comprises effecting the reaction in the presence of a solvent and contacting the glycolaldehyde with the activated catalyst.

2. The process according to claim 1, wherein the catalyst precursor comprises, as catalytically active components, one or more oxygen compounds of the metals of groups 8 and/or 9 and/or 10 and/or 11 of the periodic table of the elements.

3. The process according to at least one of claims 1 and 2, wherein the catalytically active component is an oxygen compound of Ni, Co and/or Cu.

4. The process according to at least one of claims 1 to 3, wherein the catalyst used comprises less than 0.4 mole percent of noble metal atoms selected from the group consisting of ruthenium, rhodium, palladium, silver, rhenium, osmium, iridium, platinum, gold and mercury.

5. The process according to at least one of claims 1 to 4, wherein the reaction is performed at a temperature of 15 to 350°C.

**6.** The process according to at least one of claims 1 to 5, wherein the reaction is performed at a pressure of 10 to 350 bar.

**7.** The process according to at least one of claims 1 to 6, wherein the solvent is THF or water.

**8.** The process according to at least one of claims 1 to 7, wherein the activated catalyst has a degree of reduction of 30% or more.

**9.** The process according to at least one of claims 1 to 8, wherein the activated catalyst which has been prepared by reducing a passivated catalyst has, after the activation, a degree of reduction which is at least 2% greater than the degree of reduction of the passivated catalyst.

**10.** The process according to at least one of claims 1 to 9, wherein the activated catalyst is handled under inert conditions during and after the reduction until the contacting with glycolaldehyde.

## Revendications

**1.** Procédé de mise en réaction de glycolaldéhyde avec un agent d'amination en présence d'hydrogène et d'un catalyseur, le catalyseur étant activé par réduction d'un précurseur de catalyseur ou par réduction d'un catalyseur passivé, **caractérisé en ce que** la mise en réaction a lieu en présence d'un solvant et **en ce que** le glycolaldéhyde est mis en contact avec le catalyseur activé.

**2.** Procédé selon la revendication 1, **caractérisé en ce que** le précurseur de catalyseur contient en tant que composants catalytiquement actifs un ou plusieurs composés oxygénés des métaux des groupes 8 et/ou 9 et/ou 10 et/ou 11 du tableau périodique des éléments.

**3.** Procédé selon au moins l'une quelconque des revendications 1 à 2, **caractérisé en ce que** le composant catalytiquement actif est un composé oxygéné de Ni, Co et/ou Cu.

**4.** Procédé selon au moins l'une quelconque des revendications 1 à 3, **caractérisé en ce que** le catalyseur utilisé contient moins de 0,4 pour cent en moles d'atomes de métaux nobles choisis dans le groupe constitué par le ruthénium, le rhodium, le palladium, l'argent, le rhénium, l'osmium, l'iridium, le platine, l'or et le mercure.

**5.** Procédé selon au moins l'une quelconque des revendications 1 à 4, **caractérisé en ce que** la mise en réaction est réalisée à une température de 15 à 350 °C.

**6.** Procédé selon au moins l'une quelconque des revendications 1 à 5, **caractérisé en ce que** la mise en réaction est réalisée à une pression de 10 à 350 bar.

**7.** Procédé selon au moins l'une quelconque des revendications 1 à 6, **caractérisé en ce que** le solvant est le THF ou l'eau.

**8.** Procédé selon au moins l'une quelconque des revendications 1 à 7, **caractérisé en ce que** le catalyseur activé présente un degré de réduction de 30 % et plus.

**9.** Procédé selon au moins l'une quelconque des revendications 1 à 8, **caractérisé en ce que** le catalyseur activé, qui a été fabriqué par réduction d'un catalyseur passivé, présente après l'activation un degré de réduction qui se situe au moins 2 % au-dessus du degré de réduction du catalyseur passivé.

**10.** Procédé selon au moins l'une quelconque des revendications 1 à 9, **caractérisé en ce que** le catalyseur activé est manipulé dans des conditions inertes pendant et après la réduction jusqu'à la mise en contact avec le glycolaldéhyde.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4153581 A
- US 4152353 A
- WO 03076386 A
- EP 1431271 A1
- WO 2008006750 A
- WO 2008006748 A
- WO 2008006752 A
- WO 2008006749 A
- WO 2008006754 A
- DE 2118283 A
- EP 312253 A2
- DE 3609978 A1
- US 6534441 B
- DE 4400591 A1
- US 6147261 A
- JP 3246248 B
- JP 3279342 B
- US 2009012333 A
- US 2008081931 A
- US 2007249871 A
- EP 1697291 A
- US 4503260 A
- US 4322568 A
- EP 1106600 A2
- EP 0636409 A
- EP 0742045 A
- EP 696572 A
- EP 963975 A
- EP 1317959 A


### In der Beschreibung aufgeführte Nicht-Patentliteratur


- **HOUBEN/WEYL.** Methoden der organischen Chemie. Georg Thieme Verlag Stuttgart, 1957, vol. XI/1, 602-648
- **D.F. MORROW.** *J. Med. Chem.,* 1973, vol. 16, 736-739
- **A. BEEBY ; D. B. H. MOHAMMED ; J. R. SODEAU.** *J. Am. Chem. Soc.,* 1987, vol. 109, 857-861
- **A. B. STILES.** Catalyst Manufacture - Laboratory and Commercial Preperations. Marcel Dekker, 1983
- **A. B. STILES.** Catalyst Manufacture. Marcel Dekker, Inc, 1983, 15
- Catalysis and Catalysts. **ULLMANN.** Ullmann's Encyclopedia Electronic Release. 2000, 28-32
- **ERTL ; KNÖZINGER ; WEITKAMP.** Handbook of Heterogenoeous Catalysis. VCH Weinheim, 1997, 98 ff
- **D. A. M. MONTI ; A. BAIKER.** Temperatur-Programmed Reduction. Parametric Sensitivity and Estimation of Kinetic Parameters. *J. Catal.,* 1983, vol. 83, 323-335